# EUROPEAN PATENT SPECIFICATION

(11) **EP 4 069 819 B1**
(45) Date of publication and mention of the grant of the patent: **14.02.2024**
(21) Application number: 20829795.2
(22) Date of filing: 03.12.2020
(51) Int. Cl.: C12M 1/26, C12M 1/33

(54) **DEVICE FOR THE FRAGMENTATION OF TISSUES WITHIN A SEALED STERILE ENVIRONMENT WITH AN ASEPTIC PROCEDURE AND METHOD THEREOF**
VORRICHTUNG ZUR FRAGMENTIERUNG VON GEWEBE IN EINER VERSCHLOSSENEN STERILEN UMGEBUNG MIT ASEPTISCHEM VERFAHREN UND VERFAHREN DAFÜR
DISPOSITIF POUR LA FRAGMENTATION DE TISSUS À L'INTÉRIEUR D'UN ENVIRONNEMENT STÉRILE SCELLÉ AVEC UNE PROCÉDURE ASEPTIQUE ET PROCÉDÉ ASSOCIÉ

(30) Priority: 04.12.2019 IT 201900022350
(43) Date of publication of application: 12.10.2022
(73) Proprietor: HYDRA S.r.l., 41037 Mirandola (MO) (IT)
(72) Inventor: BOSETTO, Antonio, 41037 Mirandola (Modena) (IT); PIRAZZOLI, Paolo, 41037 Mirandola (Modena) (IT)
(74) Representative: Martorana, Matteo Luca
(86) International application number: PCT/EP2020/000208
(87) International publication number: WO 2021/110282

(56) References cited:
- EP-A1- 3 106 512
- WO-A1-2016/199149
- WO-A1-2018/235102
- WO-A2-2009/047045
- WO-A2-2012/019103
- US-A- 5 396 898

## Description

### Technical Field

This invention refers to a fragmentation device and the operating method therefor for the processing of a biological material, in particular soft tissues such as adipose tissues.

### Background Art

Adipose derived stem cells (ADSCs) are mesenchymal stem cells capable of reproducing, through mitotic cell division, and differentiating into a wide range of specialised cell types.

Since these cells are abundant in the SVF (stromal vascular fraction) of adipose tissue, they are ideal as a cellular source in the field of regenerative medicine. ADSCs can be obtained from fat deposits and harvested from adipose tissue through a simple, minimally invasive harvesting procedure performed by a surgeon under general or local anaesthetic.

Recent investigations suggest that ADSCs and the stromal vascular fraction (SVF, which consists of a connective stroma whose function is to support adipocytes and blood vessels) are easily isolated from human adipose tissue through enzymatic digestion.

To obtain the ADSC and SVF, enzymatic digestion is used, which allows a large number of cells to be isolated rapidly. European legislation classifies enzymatic digestion as major manipulation and therefore it is subject to strict regulations, which greatly restrict the possibilities for clinical use. Another commonly known method consists of the isolation of tissue micro-grafts via technology involving centrifugation within a container. A grid featured in the device is used to select tissue fragments with a statistically significant portion of progenitor cells.

Other commonly known techniques may involve filtration and/or sedimentation, which may be carried out in a laboratory or during the operation comprising the removal and/or replanting of tissue.

Patent application US5396898 discloses a method of treatment of fatty tissue samples that makes use of a tubular chamber with a rotating blade, a piston pushing the treated material and a perforated base where the material is further transferred into a second container. The apparatus disclosed has no symmetric chamber for further cutting, having no blades or equivalent in the receiving chamber.

### Disclosure of Invention

The object of the invention is to develop a device and method for processing tissue fractions from biological material taken as a compact sample. The fabric of origin may be adipose tissue, or another soft biological tissue, which must be transformed into a homogeneous fluid or semi-fluid phase intended for laboratory use or for clinical application, for example, in a "one-step" operation (comprising both sample collection and reimplantation).

The invention is performed within a sealed sterile or sterilisable system to ensure the material is mechanically manipulated in aseptic conditions before being used in the laboratory or administered. In the case of adipose tissue, the product obtained from fragmentation has a higher concentration of stromal cells than the adipose tissue removed, as the latter has been homogenised and the connective fraction thereof removed.

To achieve the said object, the device comprises an insulated enclosure defined by two internal chambers interconnected by a drawing partition; in each of the said sections, a pusher piston and a cutter are configured to rotate and create micro-fragmentations of the material to be processed in conjunction with the thrust action of the relative pusher piston.

Essentially, the processing method involves loading the material to be processed into one of the two chambers or sections, activating the rotary cutter and moving the relative piston so as to transfer the material to be processed into the other section, through the said drawing partition. Subsequently, the operation of the thrust elements is reversed and the biological material is pushed through the drawing partition again, one or more times, after which it is extracted and put to its specific laboratory or clinical uses.

Another object is to provide the device with a hydraulic connection which allows the extraction or dispensing of the processed material into a syringe connected directly to the device. The syringe is filled by activating the piston for the chamber in which the hydraulic extraction connection is present.

According to one aspect of the invention, the connection is sealed with a small cap during the loading and processing phases and, at the end of the processing, the cap is removed and the sterile syringe which will house the processed material is connected in its place.

The movement of the pistons is axial and alternating, i.e. a translation movement, while the cutter movement is a flat rotation.

In accordance with one embodiment, the said cutter rotation and simultaneous advancement of the piston are achieved by means of an independent screw mechanism for each section of the device.

It is also the spirit of the invention to provide an anti-rotation device for the pistons which limits the piston to simply axial translation; in accordance with one embodiment, the said anti-rotation device is made with a runner in each of the cylinders along which pins which are integral with the pistons run. Advantageously, the pins which run in the runners also act as visual position indicators which show the piston stroke.

It is also the spirit of the invention to include a retaining device which allows the entire drawing and cutting unit to be kept assembled within a chamber or section during all the preliminary use stages, while the remaining chamber or section remains open in order to load the material to be to processed. The retaining device may be, for example, a threaded cap which features an opening to allow the gas or steam sterilisation of the processing chamber within which the drawing and cutting unit is assembled. If radiation sterilisation is used, the fact that the retaining device has openings is insignificant.

The parts of the device in contact with the biological material are made of biocompatible material.

The spirit of the invention is also to provide a device in which the components are made entirely or partly of metal or entirely or partly of plastic and which is intended to be either reusable, after washing and sterilisation, or supplied as a sterile single-use device.

Another object of the invention is to make the device operable manually through the inclusion of control knobs.

Another object of the invention is to provide a device which is operable through coupling to one or more motorised devices. Preferably, the said one or more motorised devices are coupled, directly or indirectly, to the threaded shaft which drives each pusher piston and relative rotary cutter.

Another object of the invention is to provide the motorised device with displacement sensors and electrical and mechanical safety devices capable of carrying out the operating steps for the processing of the material automatically and in a controlled manner.

According to a further object, the device is provided with one or more hydraulic connections which can be connected to an external line for a flow connection and to allow the material contained therein to be washed with a flow of saline solution, or other appropriate liquid, and in which one of the purposes of this washing step is the removal of the oily phase of the preparation and of any blood residues. Advantageously, the said one or more fluid connections are similar to that used for the extraction of the processed material.

A further object envisages that the position pins and the fluid extraction connection are aligned so that, during the extraction step, the operator can monitor the collection syringe and the position of the pistons within the processing chambers.

### Brief Description of Drawings

This and further characteristics of the invention will be better highlighted in the following description of some embodiments thereof, illustrated, in the form of a non-limiting example, with the help of accompanying drawings, in which:
Figure 1 is a sectional view of a fragmentation device within a sealed sterile environment according to the invention,

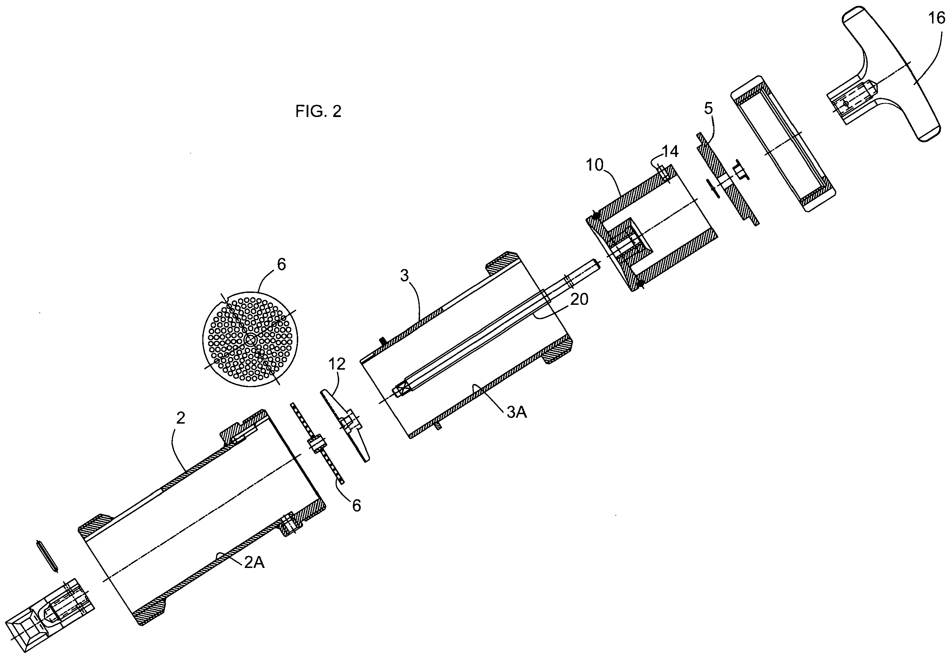

Figure 2 is an exploded configuration of some constituent elements of the device in Figure 1.

Although the present invention is described below with reference to the embodiment thereof shown in the drawings, the present invention is not limited solely to the embodiment described below and shown in the annexed drawings. On the contrary, the embodiment described and shown helps clarify certain characteristics of the present invention, whose purpose is defined by the claims.

With reference to the figures, a device as a whole for fragmenting a biological material in a sealed aseptic environment is denoted 1.

### Best Mode for Carrying Out the Invention

According to a preferred aspect of the invention, the biological material to be processed is a fragment of compact tissue, such as for example adipose tissue, from which the intention is to obtain a homogeneous fluid or semifluid mass which can be transferred into a syringe.

Essentially, the device 1 consists of two interconnected cylinders 2 and 3, sealed at the ends with caps 4 and 5, and having at least one drawing partition 6 which is held in position between the coupled cylinders, essentially in the middle of the device 1. The partition 6 is essentially a bored disk.

This way, the internal location of the drawing partition 6 defines two almost identical chambers or sections 7 and 8 interconnected by the said partition.

Each chamber or section 7, 8 houses a thrust element, such as a piston 9, 10 which is free to slide axially along the axis AA of the device 1.

The pusher pistons 9, 10 have a circular form corresponding to that of the cylinders 2 and 3 and slide within the device, guided by the relative internal walls 2A and 3A.

Each chamber or section 7, 8 also houses a rotary cutter 11, 12, preferably with multiple blades, which carries out the micro-fragmentation of the introduced material, in operational configuration. Each cutter is positioned with its cutting blade close to the partition 6 and cuts the material so that it can be extruded through the holes in the disc 6.

This way, the device is configured as a closed chamber, which is initially sterilised together with its internal parts and which allows the contents to be processed aseptically, preventing any possible external contamination, in particular that of a microbiological nature.

According to one aspect of the invention, the device 1 is made entirely or partly of re-sterilisable steel, or entirely or partly of plastic, and is intended to be either reusable, after washing and sterilisation, or supplied as a sterile single-use device.

With reference to the figures, it can be seen that the two cylinders 2 and 3 may be sealed together by means of a threaded coupling and commonly known gaskets. Preferably, the material may be loaded into one of the chambers before the coupling of the cylinders 2 and 3, carrying out the procedure within a sterile environment, such as the operating field in which the tissue harvesting is carried out; the material is dispensed, meanwhile, through a threaded outlet hole 17 provided in one of the sections and a quick connector 17A, for example a female Luer Lock (LL) connector which is standardised for the connection of syringes (which are normally equipped with a male LL connector).

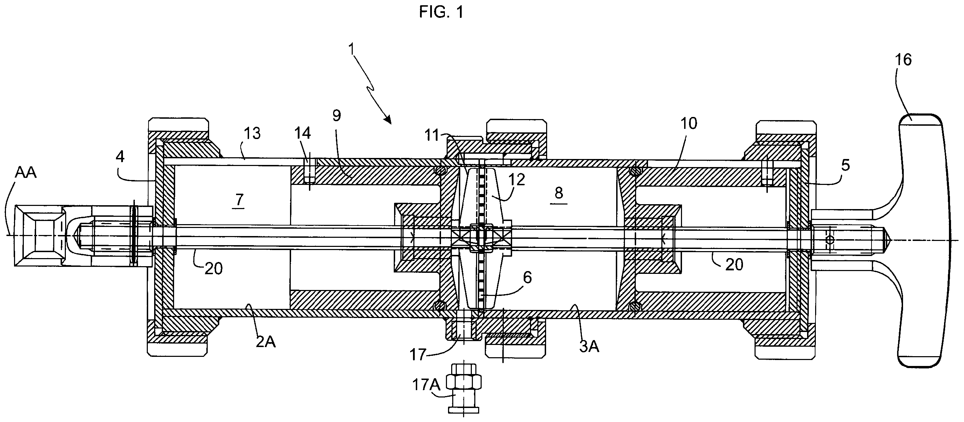

The connector 17A is normally sealed during the loading and processing phases, for example by a small sealing cap which is not shown; subsequently, the cap is removed for the extraction step and a sterile syringe is connected in its place, which receives the processed product. The extraction of the processed biological material and the simultaneous filing of the syringe takes place, once the piston 9 has been positioned at the stroke limit thereof, near the cutter 11 (as shown in Fig. 1), using the piston 10 operated by means of the control knob 16.

In one embodiment, the (translation) movement of the pistons and that of the cutters (rotation) is carried out by means of a screw mechanism 20, which is independent for the two chambers or sections.

According to one aspect of the invention there is a system to prevent rotation of the pistons, comprising a runner 13 in the cylinders 2 and 3 and pins 14 which are integral with the pistons. However, this embodiment in no way limits the way in which the required prevention action is provided, which may therefore also be provided in other ways.

Advantageously, the pins 14 in the runners also act as indicators of the stroke position of the pistons 9 and 10, to facilitate the operator.

The device 1 also comprises at least one threaded cap which allows the entire drawing and cutting unit to be kept assembled within a chamber or section, while the remaining chamber or section remains open in order to load the material to be to processed. The threaded cap features an opening to allow the gas or steam sterilisation of the processing chamber 7 and 8 within which the drawing and cutting unit is assembled. If radiation sterilisation is used, the fact that the cap has openings is insignificant.

With reference to the figures, it can be seen that the device is operable manually using at least one control knob 16.

In accordance with an embodiment not shown, the device 1 can cooperate with a motorised device (for example an electric or pneumatic device) by, for example, directly coupling the threaded shafts to appropriate mutually opposite power take-offs; advantageously, this device can be equipped with displacement sensors and appropriate electric and mechanical safety devices which carry out all the actions required of the operator in a controlled manner.

Again, and also in accordance with an embodiment not shown, one variant could envisage the presence of further fluid connections, for example, similar to those used for the extraction of the processed material, which will be connected to external lines used for washing the contents of the chambers 7 and 8 with a flow of saline solution, or other appropriate liquid, either preliminarily to, during, or following the processing. One of the purposes of this washing is to remove the blood and oily residues from the preparation, which originated during the sampling or processing steps (cell lysis).

The present invention has been described with reference to the specific embodiment shown in the illustrations; it should be noted, however, that this invention is not limited to said specific embodiment presented and described in the present document. On the contrary, further variants of the described embodiment also form part of the scope of the present invention, as detailed in the pertinent claims.

In particular, as regards the actuation of the pusher pistons, this system can be realised with different systems with respect to the screw one indicated here.

According to one aspect of the invention which is not shown, the position pins 14 may be advantageously aligned with the outlet hole 17, to facilitate the operator during the extraction step.

## Claims

1. A device (1) for the processing of a biological material, **characterised by** the fact that the said device comprises at least two cylinders (2, 3) which define chambers or sections (7, 8) which may be sealable together and interconnected by at least one drawing partition (6); in each chamber or section at least one pusher piston (9, 10) is slidable and at least one cutter (11, 12) is present; in the operational configuration, the cutter rotates and creates micro-fragmentations of the material to be processed when the said material moves from one chamber or section to the other, driven by means of the thrust action of the relative pusher piston.

2. A device (1) according to Claim 1, **characterised by** the fact that the said cutter has multiple blades.

3. A device (1) according to Claim 1, **characterised by** the fact that the said material is positioned inside one of the two chambers or sections before the device is closed.

4. A device (1) according to Claim 1, **characterised by** the fact that at least one of the chambers or sections comprises a hole (17) with fluid connector (17A) which allows the extraction, or rather the dispensing, of the material processed.

5. A device (1) according to Claim 4, **characterised by** the fact that the connector (17A) may be sealed with a cap and is normally sealed in the operational condition, during the processing step.

6. A device (1) according to Claim 1, **characterised by** the fact that the movement of the pistons is a translation movement along the axis (AA).

7. A device (1) according to Claim 1, **characterised by** the fact that the said rotation and advancement is achieved by means of an independent screw mechanism (20) for each chamber or section (7, 8).

8. A device (1) according to Claim 1, **characterised by** the fact that the said device comprises an anti-rotation device for the pistons.

9. A device (1) according to Claim 8, **characterised by** the fact that the said anti-rotation device is made with runners (13) and pins (14) therefor; the runner (13) is made in the cylinder while the pin (14) is integral with the piston, or vice versa.

10. A device (1) according to Claim 9, **characterised by** the fact that in the said anti-rotation device, the pins (14) in the runners also act as indicators of the piston stroke positions.

11. A device (1) according to at least one of the previous claims, **characterised by** the fact that the said position pins (14) are aligned with the outlet hole (17), to facilitate the operator during the extraction step.

12. A device (1) according to Claim 1, **characterised by** the fact that the said device further comprises a retaining device, such as for example a threaded cap (which may be bored) which allows the entire drawing and cutting unit in section 1 to be kept assembled during sterilisation and subsequent storage and transport of the device to the place of use, while section 2 is kept open during these steps so that it is immediately ready for the material to be processed to be loaded.

13. A device (1) according to Claim 1, **characterised by** the fact that the components thereof are made entirely or partly of metal which can be sterilised several times.

14. A device (1) according to Claim 1, **characterised by** the fact that the components thereof are made entirely or partly of plastic and may be - but not necessarily is - intended for a single use.

15. A device (1) according to Claim 1, **characterised by** the fact that the said device is operable manually by means of control knobs.

16. A device (1) according to any one of the previous claims, **characterised by** the fact that the device can be operated coupling it directly to one or more motorised devices.

17. A device (1) according to Claim 16, **characterised by** the fact that the said one or more motorised devices are coupled with the threaded shaft which drives each pusher piston and rotary cutter, through a power take-off, directly or indirectly.

18. A device (1) according to Claim 16, **characterised by** the fact that the said one or more motorised devices are equipped with displacement sensors and electric and mechanical safety devices which carry out all the actions required for the processing of the material in a controlled manner.

19. A device (1) according to any one of the previous claims, **characterised by** the fact that the device furthermore comprises one or more fluid connections which can be connected to external lines for a flow connection and to allow the contained material to be washed with a flow of saline solution, or other appropriate liquid; the said one or more fluid connections are similar to those used for the extraction of the processed material.

20. A device (1) according to any one of the previous claims, **characterised by** the fact that the said device is sterilised, sterilisable, or sterile and allows the biological material processed to be collected in a sterile container, for example a syringe, which is connected directly or indirectly to the extraction connector, in order to transfer the material into the end container with an aseptic procedure.

21. A method for processing a biological material with a device (1) according to one or more of the 17 previous claims, which includes the following steps:
a. Loading the material to be processed into one of the two chambers or sections,
b. Activating at least the cutter in the section in which the material is located,
c. Activating at least the piston for the section in which the material is and transferring the material to be processed into the other chamber or section, once it has been pushed through the drawing partition,
d. Repeating steps b) and c) one or more times,
e. Extracting the processed material.

## Patentansprüche

1. Vorrichtung (1) zum Verarbeiten von biologischem Material, **dadurch gekennzeichnet, dass** die Vorrichtung mindestens zwei Zylinder (2, 3) umfasst, die Kammern oder Sektionen (7, 8) definieren, die miteinander siegelbar sein können und durch mindestens eine Ziehunterteilung (6) miteinander verbunden sind, wobei in einer jeden Kammer oder Sektion mindestens ein Schieberkolben (9, 10) verschiebbar ist und mindestens ein Schneidwerkzeug (11, 12) vorhanden ist, wobei sich das Schneidwerkzeug in der Betriebsauslegung dreht und Mikrofragmentierungen des zu verarbeitenden Materials erzeugt, wenn sich das Material, angetrieben durch die Schubwirkung des jeweiligen Schieberkolbens, von einer Kammer oder Sektion zur anderen bewegt.

2. Vorrichtung (1) nach Anspruch 1, **dadurch gekennzeichnet, dass** das Schneidwerkzeug mehrere Messer aufweist.

3. Vorrichtung (1) nach Anspruch 1, **dadurch gekennzeichnet, dass** das Material in einer der beiden Kammern oder Sektionen positioniert wird, bevor die Vorrichtung geschlossen wird.

4. Vorrichtung (1) nach Anspruch 1, **dadurch gekennzeichnet, dass** mindestens eine der Kammern oder Sektionen ein Loch (17) mit Fluidverbinder (17A) umfasst, das das Extrahieren oder vielmehr das Ausgeben des verarbeiteten Materials erlaubt.

5. Vorrichtung (1) nach Anspruch 4, **dadurch gekennzeichnet, dass** der Verbinder (17A) mit einer Kappe versiegelt sein kann und normalerweise im Betriebszustand während des Verarbeitungsschritts versiegelt ist.

6. Vorrichtung (1) nach Anspruch 1, **dadurch gekennzeichnet, dass** die Bewegung der Kolben eine translatorische Bewegung entlang der Achse (AA) ist.

7. Vorrichtung (1) nach Anspruch 1, **dadurch gekennzeichnet, dass** die Drehung und der Vorschub mittels eines unabhängigen Schraubenmechanismus (20) für eine jede Kammer oder Sektion (7, 8) erzielt werden.

8. Vorrichtung (1) nach Anspruch 1, **dadurch gekennzeichnet, dass** die Vorrichtung eine Drehsicherungsvorrichtung für die Kolben umfasst.

9. Vorrichtung (1) nach Anspruch 8, **dadurch gekennzeichnet, dass** die Drehsicherungsvorrichtung mit Kanälen (13) und Zapfen (14) ausgebildet ist, wobei der Kanal (13) im Zylinder ausgebildet ist, während der Zapfen (14) fest mit dem Kolben verbunden ist oder umgekehrt.

10. Vorrichtung (1) nach Anspruch 9, **dadurch gekennzeichnet, dass** die Zapfen (14) in den Kanälen in der Drehsicherungsvorrichtung auch als Anzeigen für die Kolbenhubpositionen wirken.

11. Vorrichtung (1) nach mindestens einem der vorhergehenden Ansprüche, **dadurch gekennzeichnet, dass** die Positionszapfen (14) fluchtend zum Auslassloch (17) angeordnet sind, um den Bediener beim Extrahierungsschritt zu unterstützen.

12. Vorrichtung (1) nach Anspruch 1, **dadurch gekennzeichnet, dass** die Vorrichtung zudem eine Haltevorrichtung wie beispielsweise eine mit einem Gewinde versehene Kappe (die ausgebohrt sein kann) umfasst, die der gesamten Zieh- und Schneideinheit in Sektion 1 erlaubt, während des Sterilisierens und des darauffolgenden Lagerns und Transportierens der Vorrichtung zum Einsatzort montiert zu bleiben, während Sektion 2 während dieser Schritte offen gehalten wird, sodass sie sofort für das zu ladende zu verarbeitende Material bereit ist.

13. Vorrichtung (1) nach Anspruch 1, **dadurch gekennzeichnet, dass** deren Komponenten vollumfänglich oder teilweise aus Metall, das mehrmals sterilisiert werden kann, ausgebildet sind.

14. Vorrichtung (1) nach Anspruch 1, **dadurch gekennzeichnet, dass** deren Komponenten vollumfänglich oder teilweise aus Kunststoff ausgebildet sind, der für den Einmalgebrauch bestimmt sein kann, aber nicht muss.

15. Vorrichtung (1) nach Anspruch 1, **dadurch gekennzeichnet, dass** die Vorrichtung mittels Bedienknöpfen von Hand bedienbar ist.

16. Vorrichtung (1) nach einem der vorhergehenden Ansprüche, **dadurch gekennzeichnet, dass** die Vorrichtung bedient werden kann, indem sie direkt mit einer oder mehreren motorbetriebenen Vorrichtungen gekuppelt wird.

17. Vorrichtung (1) nach Anspruch 16, **dadurch gekennzeichnet, dass** die eine oder die mehreren motorbetriebenen Vorrichtungen mit dem Gewindeschaft gekuppelt sind, der einen jeden Schieberkolben und ein jedes rotatorische Schneidwerkzeug antreibt, direkt oder indirekt über einen Kraftanschluss.

18. Vorrichtung (1) nach Anspruch 16, **dadurch gekennzeichnet, dass** die eine oder die mehreren motorbetriebenen Vorrichtungen mit Wegsensoren sowie elektrischen und mechanischen Sicherheitseinrichtungen ausgestattet sind, die alle Vorgänge durchführen, die notwendig sind, um das Material auf kontrollierte Weise zu verarbeiten.

19. Vorrichtung (1) nach einem der vorhergehenden Ansprüche, **dadurch gekennzeichnet, dass** die Vorrichtung zudem eine oder mehrere Fluidverbindungen umfasst, die mit externen Leitungen für eine Strömungsverbindung verbunden werden können, und um dem enthaltenen Material zu erlauben, mit einem Strom Salzlösung oder einer anderen geeigneten Flüssigkeit gewaschen zu werden, wobei die eine oder die mehreren Fluidverbindungen denen ähneln, die zum Extrahieren des verarbeiteten Materials genutzt werden.

20. Vorrichtung (1) nach einem der vorhergehenden Ansprüche, **dadurch gekennzeichnet, dass** die Vorrichtung sterilisiert, sterilisierbar oder steril ist und dem verarbeiteten biologischen Material erlaubt, in einem sterilen Behälter, beispielsweise einer Spritze, gesammelt zu werden, der direkt oder indirekt mit dem Extraktionsverbinder verbunden ist, um das Material mittels eines aseptischen Verfahrens in den Endbehälter zu transferieren.

21. Verfahren zum Verarbeiten von biologischem Material mit einer Vorrichtung (1) nach einem oder mehreren der 17 vorhergehenden Ansprüche, das die folgenden Schritte einschließt:
a. Laden des zu verarbeitenden Materials in eine der beiden Kammern oder Sektionen;
b. Aktivieren von mindestens dem Schneidwerkzeug in der Sektion, in die das Material geladen wurde;
c. Aktivieren von mindestens dem Kolben für die Sektion, in der sich das Material befindet, und Transferieren des zu verarbeitenden Materials in die andere Kammer oder Sektion, wenn dieses durch die Ziehunterteilung geschoben wurde;
d. Wiederholen der Schritte b) und c) einmal oder mehrere Male;
e. Extrahieren des verarbeiteten Materials.

## Revendications

1. Un dispositif (1) pour le traitement de matériau biologique, **caractérisé par le fait que** ledit dispositif comprend au moins deux cylindres (2, 3) qui définissent deux chambres ou sections (7, 8), qui peuvent être scellées ensemble et interconnectées par au moins une cloison de tirage (6) ; dans chaque chambre ou section se trouvent au moins un piston de poussée (9, 10) coulissant et au moins un coupeur (11,12); dans la configuration opérationnelle, le coupeur tourne et crée des micro fragments de matériau à traiter, lorsque ledit matériau se déplace d'une chambre ou section à l'autre, entraîné par l'action de poussée du piston de poussée relatif.

2. Un dispositif (1) conformément à la Revendication1, **caractérisé par le fait que** ledit coupeur a plusieurs lames.

3. Un dispositif (1) conformément à la Revendication 1 **caractérisé par le fait que** ledit matériau est placé dans une ou deux chambres ou sections avant de refermer le dispositif.

4. Un dispositif (1) conformément à la Revendication 1 **caractérisé par le fait qu'**au moins une des chambres ou sections comprend un orifice (17) avec un raccord de fluide permettant d'extraire ou plutôt de distribuer le matériau traité.

5. Un dispositif (1) conformément à la Revendication 4, **caractérisé par le fait que** le raccord (17A) peut être scellé avec un capuchon et qu'il est normalement scellé en condition opérationnelle pendant la phase de traitement.

6. Un dispositif (1) conformément à la Revendication 1, **caractérisé par le fait que** le mouvement des pistons est un mouvement de translation le long de l'axe (AA).

7. Un dispositif (1) conformément à la Revendication 1 **caractérisé par le fait que** ladite rotation et le dit avancement sont obtenus par un mécanisme de vis indépendant (20) pour chaque chambre ou section (7,8).

8. Un dispositif (1) conformément à la Revendication1, **caractérisé par le fait que** ledit dispositif comprend un dispositif antirotation pour les pistons.

9. Un dispositif (1) conformément à la Revendication 8, **caractérisé par le fait que** ledit dispositif antirotation est donc fait de patins (13) et de broches (14) ; le patin (13) est fait dans le cylindre tandis que la broche (14) est intégrée au piston ou vice-versa.

10. Un dispositif (1) conformément à la Revendication 9 **caractérisé par le fait que** ledit dispositif antirotation, les broches (14) et les patins font aussi office d'indicateurs de position de la course du piston.

11. Un dispositif (1) conformément à au moins une des revendications précédentes **caractérisé par le fait que** lesdites broches de position (14) sont alignées avec l'orifice de sortie (17) en vue de faciliter l'opérateur pendant la phase d'extraction.

12. Un dispositif (1) conformément à la Revendication 1, **caractérisé par le fait que** ledit dispositif comprend aussi un dispositif de retenue, tel que par exemple un capuchon fileté (qui peut être creusé) permettant à tout le groupe de tirage et de découpe dans la section 1 d'être maintenu assemblé pendant la stérilisation, le stockage et transport successifs du dispositif vers le lieu d'utilisation, tandis que la section 2 est maintenue ouverte pendant ces phases de façon à être immédiatement prête pour charger et traiter le matériau.

13. Un dispositif (11) conformément à la Revendication 1 **caractérisé par le fait que** les composants sont entièrement ou en partie en métal, que l'on peut stériliser plusieurs fois.

14. Un dispositif (11) conformément à la Revendication 1 **caractérisé par le fait que** les composants sont entièrement ou en partie en plastique, que l'on peut, mais pas nécessairement, considérer comme à usage unique.

15. Un dispositif (1) conformément à la Revendication1, **caractérisé par le fait que** ledit dispositif peut être actionné manuellement à l'aide de molettes de contrôle.

16. Un dispositif (1) conformément à l'une des revendications précédentes **caractérisé par le fait que** le dispositif peut être actionné en l'accouplant directement à un ou plusieurs dispositifs motorisés.

17. Un dispositif (1) conformément à la Revendication 16 **caractérisé par le fait que** ledit ou lesdits dispositif/s motorisé/s est/sont accouplé/s à l'arbre fileté qui entraîne le piston de poussée et le coupeur rotatif, à travers une prise de force directement ou indirectement.

18. Un dispositif (1) conformément à la Revendication 16 **caractérisé par le fait que** ledit/lesdits dispositif/s motorisé/s est/sont muni/s de capteurs de mouvement et de dispositif de sécurité électrique et mécanique qui peuvent accomplir toute les actions exigées pour traiter le matériau de façon contrôlée.

19. Un dispositif (1) conformément à l'une des revendications ci-dessus, **caractérisé par le fait que** le dispositif comprend aussi un ou plusieurs raccords de fluide qui peuvent être raccordés sur des lignes externes de circulation d'une solution saline ou d'un autre liquide approprié ; ledit/lesdits raccord/s de fluide est(sont similaire/s à celui/ceux utilisé/s pour extraire le matériau traité.

20. Un dispositif (1) conformément à l'une des revendications précédentes, **caractérisé par le fait que** ledit dispositif est stérilisé, stérilisable ou stérile et permet au matériau biologique traité d'être recueilli dans un récipient stérile, par exemple une seringue, qui es raccordée directement ou indirectement sur le raccord d'extraction, en vue de transférer le matériau dans le récipient final suivant une procédure aseptique.

21. Une méthode de traitement de matériau biologique avec un dispositif (1) conformément à une ou plusieurs des 17 revendications précédentes, comprenant les phases suivantes :
a. Le chargement du matériau à traiter dans une ou deux chambres ou sections,
b. Activation d'au moins le coupeur dans la section dans laquelle le matériau est situé,
c. Activation d'au moins le piston de la section dans laquelle se trouve le matériau et transfert du matériau à traiter dans l'autre chambre ou section, une fois qu'il a été poussé à travers la cloison de tirage,
d. Répétition des phases b) et c) une ou plusieurs fois,
e. Extraction du matériau traité.
